# EUROPÄISCHE PATENTANMELDUNG

(11) **EP 4 512 433 A1**
(43) Veröffentlichungstag der Anmeldung: **26.02.2025**
(21) Anmeldenummer: 24169487.6
(22) Anmeldetag: 10.04.2024
(51) Int. Cl.: A61L 2/18

(54) **VERFAHREN ZUM STEUERN EINER STERILISATIONSMASCHINE FÜR BEHÄLTER, STERILISATIONSMASCHINE UND ANLAGE**

(30) Priorität: 20.04.2023 DE 102023110021
(71) Anmelder: KHS GmbH, 44143 Dortmund (DE)
(72) Erfinder: Leyendecker, Jan, 44143 Dortmund (DE)

(57) **Zusammenfassung**

Die Erfindung betrifft ein Verfahren (100) zum Steuern einer Sterilisationsmaschine (10) für Behälter, wobei die Sterilisationsmaschine (10) mindestens eine Mischvorrichtung (14) zum Erzeugen eines Sterilisationsgemisches aufweist und mindestens einen ersten Zustand, in dem das Sterilisationsgemisch in zu sterilisierende Behälter eingeleitet wird oder eingeleitet werden soll, und einen zweiten Zustand, in dem die Einleitung des Sterilisationsgemisches in die Behälter unterbrochen ist, umfasst, wobei im ersten Zustand ein Sterilisationsmittelstrom und ein Trägerfluidstrom zum Erzeugen des Sterilisationsgemisches in die Mischvorrichtung (14) eingeleitet (102) werden und das Sterilisationsgemisch den zu sterilisierenden Behältern zugeführt wird, wobei zum Wechseln in den zweiten Zustand der Sterilisationsmittelstroms abgeschaltet (104) wird, während der Trägerfluidstrom im zweiten Zustand weiterströmt. Damit stellt die Erfindung ein Verfahren (100) zum Steuern einer Sterilisationsmaschine (10) für Behälter bereit, das kostengünstig und bedienerfreundlich ist.

## Beschreibung

Die Erfindung betrifft ein Verfahren zum Steuern einer Sterilisationsmaschine für Behälter, eine Sterilisationsmaschine und eine Anlage.

Bei der Herstellung und/oder Befüllung von Behältern, insbesondere von Kunststoffbehältern, in denen Lebensmittel und/oder Getränke gelagert werden sollen, werden die Behälter verschiedenen Behandlungen unterzogen, um die Behälter zum Beispiel zu sterilisieren. Dazu können zum Beispiel Flüssigkeiten oder Gase in die Behälter eingeleitet werden. Um ein Fluid in die Behälter einzuleiten, werden Vorrichtungen zum Verteilen von Fluid in die Behälter verwendet. Bei einem Wechsel von Betriebszuständen ist es günstig, wenn die Parameter für die in die Behälter eingeleiteten Fluide stabil bleiben. Es ist daher günstig einen konstanten Volumenstrom für die Zuführung des Fluids herzustellen, der auch bei Unterbrechungen der Behandlung der Behälter konstant verbleibt.

Dazu ist aus EP 2604295 A1 bekannt, bei Betriebsstörungen der Behandlungsanlage das Fluid über einen Bypass abzuleiten und nicht in die Behälter gelangen zu lassen. Der Fluidzustrom kann bei Betriebsstörungen konstant gehalten werden. Allerdings ist diese Anlage komplex aufgebaut und bedarf einer komplexen Steuerung.

Aufgabe der Erfindung ist es, ein Verfahren zum Steuern einer Sterilisationsmaschine für Behälter sowie eine Sterilisationsmaschine bereitzustellen, die kostengünstig und bedienerfreundlich sind.

Die Aufgabe wird durch die Merkmale der unabhängigen Ansprüche gelöst. Vorteilhafte Weiterbildungen sind Gegenstand der abhängigen Ansprüche sowie der folgenden Beschreibung.

Bei einem Verfahren zum Steuern einer Sterilisationsmaschine für Behälter, wobei die Sterilisationsmaschine mindestens eine Mischvorrichtung zum Erzeugen eines Sterilisationsgemisches aufweist und mindestens einen ersten Zustand, in dem das Sterilisationsgemisch in zu sterilisierende Behälter eingeleitet wird oder eingeleitet werden soll, und einen zweiten Zustand, in dem die Einleitung des Sterilisationsgemisches in die Behälter unterbrochen ist, umfasst, wobei im ersten Zustand ein Sterilisationsmittelstrom und ein Trägerfluidstrom zum Erzeugen des Sterilisationsgemisches in die Mischvorrichtung eingeleitet werden und das Sterilisationsgemisch den zu sterilisierenden Behältern zugeführt wird, ist gemäß der Erfindung vorgesehen, dass zum Wechseln in den zweiten Zustand der Sterilisationsmittelstrom abgeschaltet wird, während der Trägerfluidstrom im zweiten Zustand weiterströmt.

Mit der Erfindung wird ein Verfahren zum Steuern einer Sterilisationsmaschine für Behälter bereitgestellt, bei dem bei Behandlungsunterbrechungen oder Produktionsausfällen eine Abschaltung des Sterilisationsmittelstroms erfolgen kann. Bei Behandlungsunterbrechungen oder Produktionsausfällen wird damit die Einleitung des Sterilisationsgemisches in die Behälter unterbrochen, ohne den Trägerfluidstrom zu unterbrechen. Die Sterilisationsmaschine kann bei Behandlungsunterbrechungen oder Produktionsausfällen in den zweiten Zustand überführt werden, indem der Sterilisationsmittelstrom abgeschaltet ist. Durch das Abschalten des Sterilisationsmittelstroms wird in der Mischvorrichtung kein Sterilisationsgemisch mehr hergestellt. Der Trägerfluidstrom wird dabei nicht abgeschaltet, sodass seine Parameter unverändert bleiben können. Unabhängig davon, wohin der Trägerfluidstrom abgeleitet wird, wird kein weiteres Sterilisationsmittel mit ihm abgeleitet. Wenn zum Beispiel der Trägerfluidstrom in eine Umgebung der Sterilisationsmaschine abgeleitet wird, erhöht sich die Konzentration des Sterilisationsmittels in der Umgebung nicht. Falls Wartungsarbeiten an der Sterilisationsmaschine durchgeführt werden müssen, kann Bedienpersonal den Raum um die Sterilisationsmaschine daher früher als im Stand der Technik betreten, da Ausfallzeiten wegen Wartung bzw. Behandlungsunterbrechungen reduziert werden können. Dies reduziert die Kosten bzw. erhöht die Produktivität der Sterilisationsmaschine und ggf. einer Anlage zum Herstellen und/oder Befüllen von Behältern, deren Teil die Sterilisationsmaschine sein kann. Weiter wird dadurch die Bedienung der Sterilisationsmaschine vereinfacht, da Wartezeiten vor dem Betreten des Raums, in dem die Sterilisationsmaschine angeordnet ist, reduziert werden können bzw. gegebenenfalls vollständig auf Wartezeiten verzichtet werden kann.

Behälter im Sinne der Erfindung können beispielsweise Flaschen, insbesondere Getränkeflaschen, oder Vorformlinge zum (Streck-)Blasen von Flaschen sein. Die Behälter können z. B. aus einem Kunststoffmaterial, insbesondere PET, bestehen.

In einem Ausführungsbeispiel können eine Temperatur, ein Druck und/oder ein Volumenstrom des Trägerfluidstroms im zweiten Zustand gegenüber dem ersten Zustand unverändert oder im Wesentlichen unverändert bleiben. Unter einer im Wesentlichen unveränderten Abweichung wird eine Abweichung von höchstens +/- 10%, vorzugsweise höchstens +/- 5%, verstanden. Wenn diese Parameter im zweiten Zustand unverändert oder im Wesentlichen unverändert bleiben, kann eine schnelle Prozessstabilisierung erfolgen.

Gemäß einem Ausführungsbeispiel kann die Sterilisationsmaschine mindestens ein Ventil für das Einleiten des Sterilisationsmittelstroms aufweisen, wobei das Ventil beim Übergang in den zweiten Zustand geschlossen wird. Alternativ oder zusätzlich kann eine Pumpe abgeschaltet werden, die den Sterilisationsmittelstrom erzeugt.

Das Ventil kann dabei in einer Leitung angeordnet sein, über die der Sterilisationsmittelstrom der Sterilisationsmaschine bzw. der Mischvorrichtung zugeführt werden kann. Durch das Schließen des Ventils wird der Sterilisationsmittelstrom unterbrochen. Das Ventil bzw. ein Aktuator des Ventils kann vorzugsweise durch eine Steuereinheit bzw. eine Steuervorrichtung angesteuert werden, um geschaltet zu werden, beispielsweise um zu öffnen bzw. zu schließen.

Gemäß einem weiteren Ausführungsbeispiel kann der Sterilisationsmittelstrom Wasserstoffperoxid sein oder Wasserstoffperoxid aufweisen.

Es ist zum Beispiel denkbar, dass der Trägerfluidstrom im zweiten Zustand anstatt in die zu sterilisierenden Behälter in eine Bypassleitung eingeleitet wird. Da der Trägerfluidstrom in der Regel eine erhöhte Temperatur als die Umgebungstemperatur aufweist, kann damit eine Weiterbehandlung der Behälter mit den Trägerfluidstrom vermieden werden. Insbesondere kann eine Schrumpfung der Behälter vermieden werden.

Weiter kann der Trägerfluidstrom im zweiten Zustand zum Beispiel stromaufwärts vor der Sterilisationsmaschine umgeleitet werden. Der Trägerfluidstrom kann in diesem Ausführungsbeispiel bereits umgeleitet werden, bevor er in die Sterilisationsmaschine bzw. die Mischvorrichtung eintritt. Der Begriff stromaufwärts bezieht sich dabei auf die Fließrichtung des Trägerfluidstroms.

Gemäß einem weiteren Ausführungsbeispiel kann der Trägerfluidstrom im zweiten Zustand stromaufwärts vor dem Einleiten in die Behälter in der Sterilisationsmaschine abgesaugt werden. In diesem Ausführungsbeispiel kann der Trägerfluidstrom daher in die Sterilisationsmaschine eintreten und wird abgesaugt, bevor er in die Behälter eingeleitet wird.

Weiter ist beispielsweise denkbar, dass der Trägerfluidstrom im zweiten Zustand in einen die Sterilisationsmaschine umgebenden Isolatorraum ausströmt. Der Isolatorraum kann dazu dienen, das während der Sterilisation der Behälter aus der Sterilisationsmaschine austretende Sterilisationsmittel von einer Umgebung des Isolatorraums abzuschirmen bzw. das ausgetretene Sterilisationsmittel in dem Isolatorraum einzufangen und aus der Luft zu filtern.

Gemäß einem weiteren Ausführungsbeispiel kann der Trägerfluidstrom erwärmte Luft sein oder erwärmte Luft aufweisen.

Die erwärmte Luft kann als Träger für das Sterilisationsmittel dienen, das in der Mischvorrichtung dem Trägerfluidstrom beigemischt wird. Weiter kann die erwärmte Luft eine Temperatur von mindestens 40 °C, vorzugsweise mindestens 60 °C, aufweisen. Die Höchsttemperatur kann durch die Glastemperatur oder Schmelztemperatur des Materials der Vorformlinge bzw. Behälter definiert sein.

Falls es sich bei den Behältern um mit einem Füllgut zu befüllende Flaschen handelt, erfolgt die Sterilisation der Behälter mittels der Sterilisationsmaschine vorzugsweise vor dem Abfüllvorgang. Handelt es sich bei den Behältern um Vorformlinge zum (Streck-)Blasen von Flaschen, erfolgt die Sterilisation der Behälter mittels der Sterilisationsmaschine, vorzugsweise vor dem (Streck-)Blasprozess.

Weiter betrifft die Erfindung eine Sterilisationsmaschine für Behälter, umfassend mindestens eine Mischvorrichtung zum Erzeugen eines Sterilisationsgemisches und mindestens eine Steuervorrichtung zum Steuern der Sterilisationsmaschine, wobei die Sterilisationsmaschine mindestens einen ersten Zustand, in dem das Sterilisationsgemisch in zu sterilisierende Behälter eingeleitet wird oder eingeleitet werden soll, und einen zweiten Zustand, in dem die Einleitung des Sterilisationsgemisches in die Behälter unterbrochen ist, aufweist, wobei die Steuervorrichtung erfindungsgemäß zum Durchführen des Verfahrens nach der vorhergehenden Beschreibung ausgebildet ist und der Sterilisationsmittelstrom im zweiten Zustand abgeschaltet ist, während der Trägerfluidstrom weiterströmt.

Vorteile und Wirkungen sowie Weiterbildungen der Sterilisationsmaschine ergeben sich aus den Vorteilen und Wirkungen sowie Weiterbildungen des oben beschriebenen Verfahrens. Zur Vermeidung von Wiederholungen wird daher in dieser Hinsicht auf die vorangegangene Beschreibung verwiesen.

In diesem Aspekt der Erfindung ist die Steuervorrichtung ein Teil der Sterilisationsmaschine. Die Steuereinrichtung kann weiter dazu ausgebildet sein, Steuersignale von einer übergeordneten Steuereinheit zu empfangen, zum Beispiel von einer Anlage zur Herstellung und/oder Befüllung von Behältern.

Gemäß einem Ausführungsbeispiel kann die Sterilisationsmaschine mindestens ein Ventil zum Steuern des Sterilisationsmittelstroms aufweisen, wobei die Steuervorrichtung zum Übermitteln eines Steuersignals zum Schalten des Ventils an einen Aktuator des Ventils ausgebildet sein kann.

Das Ventil kann in einer Zuleitung zu der Mischvorrichtung angeordnet sein, um die Einleitung des Sterilisationsmittelstroms in die Mischvorrichtung zu unterbrechen oder den Sterilisationsmittelstrom umzuleiten, z. B. in einen Vorlagebehälter des Sterilisationsmittels, bevor der Sterilisationsmittelstrom die Mischvorrichtung erreicht. Alternativ oder zusätzlich kann eine Pumpe abgeschaltet werden, die den Sterilisationsmittelstrom erzeugt. Das Ventil kann dazu ausgebildet sein, Steuersignale kabelgebunden oder kabellos zu empfangen. Alternativ oder zusätzlich kann der Sterilisationsmittelstrom vor dem Einleiten in die Mischvorrichtung auch umgeleitet werden, zum Beispiel in einen Sammelbehälter.

In einem weiteren Aspekt betrifft die Erfindung eine Anlage zum Herstellen und/oder Befüllen von Behältern, umfassend mindestens eine Verarbeitungsmaschine, mindestens eine Transportvorrichtung, mindestens eine Steuereinheit und mindestens eine Sterilisationsmaschine, wobei die Sterilisationsmaschine mindestens eine Mischvorrichtung zum Erzeugen eines Sterilisationsgemisches und mindestens einen ersten Zustand, in dem ein Sterilisationsgemisch in zu sterilisierende Behälter eingeleitet wird oder eingeleitet werden soll, und einen zweiten Zustand, in dem die Einleitung des Sterilisationsgemisches in die Behälter unterbrochen ist, aufweist, wobei die Transportvorrichtung zum Transportieren von zu sterilisierenden Behältern von der Verarbeitungsmaschine zur Sterilisationsmaschine oder zum Transportieren von mittels der Sterilisationsmaschine sterilisierter Behälter zu der Verarbeitungsmaschine ausgebildet ist, wobei die Steuereinheit erfindungsgemäß zum Steuern zumindest der Sterilisationsmaschine gemäß dem Verfahren nach der vorangegangenen Beschreibung ausgebildet ist und der Sterilisationsmittelstrom im zweiten Zustand abgeschaltet ist, während der Trägerfluidstrom weiterströmt.

Die Steuereinheit kann dabei Teil der Sterilisationsmaschine sein. Alternativ kann die Steuereinheit außerhalb der Sterilisationsmaschine angeordnet sein und zusätzlich zum Steuern weiterer Maschinen ausgebildet sein, wie zum Beispiel der Behälterherstellungsmaschine.

Gemäß einem Ausführungsbeispiel kann die Verarbeitungsmaschine eine Behälterherstellungsmaschine, insbesondere eine Blas- oder Streckblasmaschine, sein, wobei die Transportvorrichtung die Behälter von der Verarbeitungsmaschine zur Sterilisationsmaschine transportieren kann.

Gemäß einem anderen Ausführungsbeispiel kann die Verarbeitungsmaschine eine Abfüllmaschine, insbesondere eine Getränkeabfüllmaschine, sein, wobei die Transportvorrichtung die Behälter von der Sterilisationsmaschine zur Verarbeitungsmaschine transportieren kann.

Gemäß einem Ausführungsbeispiel kann die Anlage mindestens ein Ventil für das Einleiten des Sterilisationsmittelstroms aufweisen, wobei die Steuereinheit zum Übermitteln eines Steuersignals zum Schalten des Ventils an einen Aktuator des Ventils ausgebildet ist. Das Schalten des Ventils kann zum Beispiel ein Öffnen bzw. Schließen des Ventils oder ein Umschalten des Ventils bewirken. Das Ventil kann gemäß den oben angeführten Erläuterungen zur Sterilisationsmaschine ausgebildet sein.

Weiter ist zum Beispiel denkbar, dass die Sterilisationsmaschine in einem Isolatorraum der Anlage angeordnet ist.

Die Transportvorrichtung kann die von der Behälterherstellungsmaschine hergestellten Behälter in den Isolatorraum hinein transportieren, um sie dort der Sterilisationsmaschine zu übergeben.

Weitere Vorteile und Wirkungen sowie Weiterbildungen der Anlage ergeben sich aus den Vorteilen und Wirkungen sowie Weiterbildungen der oben beschriebenen Sterilisationsmaschine sowie des oben beschriebenen Verfahrens. Zur Vermeidung von Wiederholungen wird daher in dieser Hinsicht auf die vorangegangene Beschreibung verwiesen.

Im Folgenden wird die Erfindung anhand beispielhafter Ausführungsformen mittels der beigefügten Zeichnung beschrieben. Es zeigen:
- Figur 1: eine schematische Darstellung der Sterilisationsmaschine;
- Figur 2: eine schematische Darstellung einer Anlage zum Herstellen von Behältern; und
- Figur 3: ein Flussdiagramm des Verfahrens zum Steuern einer Sterilisationsmaschine für Behälter.

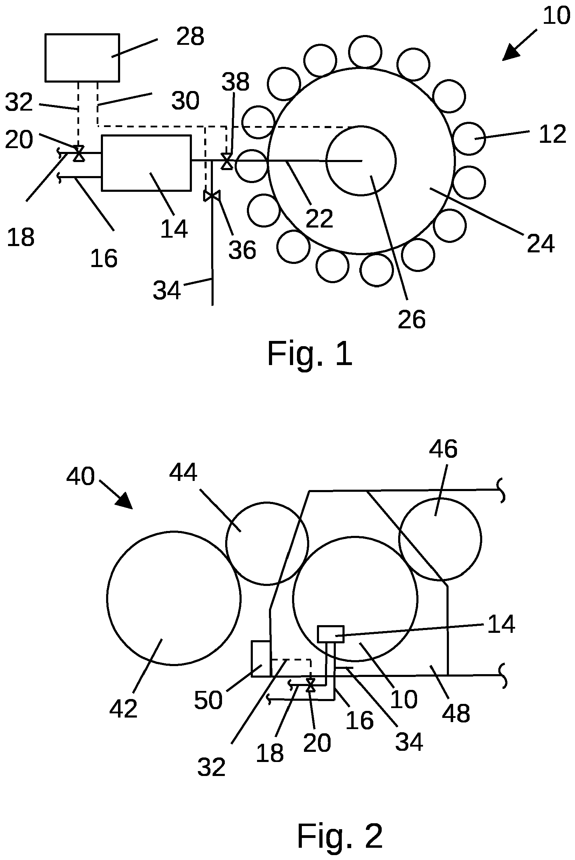

Gemäß Figur 1 wird eine beispielhafte Sterilisationsmaschine in ihrer Gesamtheit mit dem Bezugszeichen 10 referenziert.

Die Sterilisationsmaschine 10 weist eine Mischvorrichtung 14 auf, die eine erste Zuleitung 16 und eine zweite Zuleitung 18 aufweist. Über die erste Zuleitung 16 kann der Mischvorrichtung 14 ein Trägerfluidstrom zugeführt werden, der beispielsweise erwärmte Luft sein kann oder erwärmte Luft aufweisen kann. Über die zweite Zuleitung 18 kann der Mischvorrichtung 14 ein Sterilisationsmittelstrom zugeführt werden, der beispielsweise Wasserstoffperoxid sein kann oder Wasserstoffperoxid aufweisen kann. In der Mischvorrichtung 14 wird der Sterilisationsmittelstrom mit den Trägerfluidstrom gemischt, um ein Sterilisationsgemisch zu erhalten. Das Sterilisationsgemisch kann über eine dritte Zuleitung 22 einer Verteilerstation 26 der Sterilisationsmaschine 10 zugeführt werden.

Die Sterilisationsmaschine 10 kann diesem Ausführungsbeispiel weiter eine Vielzahl von Sterilisationsstationen 12 aufweisen, die an einem drehbar gelagerten Transportrad 24 verteilt angeordnet sein können. Die Sterilisationsstationen 12 können dazu an einem Umfang des Transportrads 24 äquidistant verteilt angeordnet sein. Weiter können die Sterilisationsstationen 12 dazu ausgebildet sein, zu sterilisierende Behälter aufzunehmen und mit einem Sterilisationsgemisch zu beaufschlagen.

Über die Verteilerstation 26 kann das Sterilisationsgemisch den Sterilisationsstationen 12 zum Sterilisieren der von den Sterilisationsstationen 12 aufgenommenen Behälter zugeführt werden. Das Sterilisationsgemisch kann dazu in die Behälter eingeleitet werden. Nach und während der Sterilisation kann das Sterilisationsgemisch aus den Behältern in den die Sterilisationsmaschine 10 umgebenden Raum strömen, aus dem es abgesaugt oder in anderer Weise gefiltert werden kann.

Die zweite Zuleitung 18 kann ein erstes Ventil 20 aufweisen, mit dem die zweite Zuleitung 18 geschlossen und geöffnet werden kann, um den Sterilisationsmittelstrom abzuschalten bzw. einzuschalten.

Weiter kann Sterilisationsmaschine 10 eine Steuervorrichtung 28 aufweisen, die über eine Signalverbindung 32 mit dem ersten Ventil 20 bzw. mit einem Aktuator des ersten Ventils 20 verbunden ist. Die Signalverbindung 32 kann kabelgebunden oder kabellos ausgebildet sein.

Über die Signalverbindung 32 kann die Steuervorrichtung 28 ein Schaltsignal an das erste Ventil 20 übermitteln, um das erste Ventil 20 zu schalten, z. B. um es zu öffnen oder zu schließen oder umzuschalten. Damit kann die Steuervorrichtung 28 den Sterilisationsmittelstrom ein- und ausschalten.

Weiter kann die Sterilisationsmaschine 10 eine Bypassleitung 34 aufweisen, die in diesem Ausführungsbeispiel von der dritten Zuleitung 22 abzweigen kann. Mittels eines zweiten Ventils 36, mit dem die Bypassleitung 34 zur dritten Zuleitung geöffnet und geschlossen werden kann, und eines dritten Ventils 38, mit dem die dritte Zuleitung 22 zwischen der Bypassleitung 34 und der Verteilerstation 26 geöffnet und geschlossen werden kann, kann ein aus der Mischvorrichtung 14 austretender Fluidstrom zwischen der dritten Zuleitung 22 und der Bypassleitung 34 umgeleitet werden.

Die Steuervorrichtung 28 kann über weitere Signalverbindungen 30 das zweite Ventil 36 und das dritte Ventil 38 steuern.

Die Sterilisationsmaschine 10 weist mindestens zwei Zustände auf. In einem ersten Zustand wird das Sterilisationsgemisch in zu sterilisieren Behälter eingeleitet, die von den Sterilisationsstationen 12 aufgenommen sein können. Dazu kann die Mischvorrichtung 14 aus dem Trägerfluidstrom und dem Sterilisationsmittelstrom das Sterilisationsgemisch erzeugen und über die dritte Zuleitung 22 der Verteilerstation 26 zuführen. Das dritte Ventil 38 kann dazu geöffnet sein und das zweite Ventil 36 kann dazu geschlossen sein. Weiter kann das erste Ventil 20 dazu geöffnet sein.

In einem zweiten Zustand der Sterilisationsmaschine 10 ist, beispielsweise aufgrund einer Produktionsunterbrechung der Behälter oder einer Betriebsstörung der Sterilisationsmaschine, die Einleitung des Sterilisationsgemisches in die Behälter unterbrochen. Dazu ist im zweiten Zustand der Sterilisationsmittelstrom abgeschaltet, zum Beispiel durch das Schließen des ersten Ventils 20. Alternativ kann eine Pumpe (nicht dargestellt) abgeschaltet werden, die den Sterilisationsmittelstrom erzeugt.

Im zweiten Zustand strömt der Trägerfluidstrom weiter. Dazu kann der Trägerfluidstrom weiterhin durch die Mischvorrichtung 14 in die Zuleitung 22 strömen. Da die Mischvorrichtung 14 aufgrund der Abschaltung des Sterilisationsmittelstroms kein Sterilisationsgemisch erzeugen kann, kann im zweiten Zustand lediglich das Trägerfluid durch die dritte Zuleitung 22 in Richtung der Verteilerstation 26 strömen.

Gemäß einem ersten Ausführungsbeispiel kann das Trägerfluid weiterhin in die Behälter eingeleitet werden, die von den Sterilisationsstationen 12 aufgenommen sind.

Gemäß einem zweiten Ausführungsbeispiel kann der Trägerfluidstrom durch das Schließen des dritten Ventils 38 und das Öffnen des zweiten Ventils 36 in die Bypassleitung 34 umgeleitet werden. Aus der Bypassleitung 34 kann der Trägerfluidstrom dann in eine Umgebung der Sterilisationsmaschine 10 eingeleitet werden, zum Beispiel in einen Isolatorraum, in dem sich die Sterilisationsmaschinen 10 befindet.

Gemäß einem dritten Ausführungsbeispiel kann der Trägerfluidstrom vor dem Einleiten in die Behälter, zum Beispiel an der dritten Zuleitung 22 oder an der Verteilerstation 26, mit einer Absaugvorrichtung (nicht dargestellt) abgesaugt werden.

Die oben beschriebene Sterilisationsmaschine 10 kann Teil einer Anlage 40 sein, die weiter mindestens eine Behälterherstellungsmaschine 42, mindestens eine Transport 44 und mindestens eine Steuereinheit 50 aufweist.

In Figur 2 ist beispielhaft eine Anlage 40 dargestellt, die jedoch ein anderes Ausführungsbeispiel der Sterilisationsmaschine 10 aufweist, die auch unabhängig von der Anlage 40 sein kann.

In diesem Ausführungsbeispiel der Sterilisationsmaschine 10 zweigt die Bypassleitung 34 von der ersten Zuleitung 16 ab. Eine Umleitung des Trägerfluidstroms kann daher vor dem Eintritt des Trägerfluidstroms in die Sterilisationsmaschine 10 bzw. die Mischvorrichtung 14 erfolgen. Der Auslass der Bypassleitung 34 kann in dem Isolatorraum 48 angeordnet sein. Alternativ kann der Auslass der Bypassleitung 34 auch außerhalb des Isolatorraums 48 angeordnet sein. Weiter kann auch in diesem Ausführungsbeispiel alternativ oder zusätzlich eine Absaugung des Trägerfluidstroms erfolgen, wenn die Sterilisationsmaschine 10 im zweiten Zustand ist.

Die Behälterherstellungsmaschine 42 kann zum Beispiel Kunststoffbehälter herstellen, die von der Transportvorrichtung 44, die ein drehbar gelagertes Transportrad aufweisen kann, in einen Isolatorraum 48 transportiert werden. In dem Isolatorraum 48 kann die Sterilisationsmaschine 10 angeordnet sein, der die zu sterilisierenden Behälter von der Transportvorrichtung 44 übergeben werden.

Weiter kann die Anlage 40 eine weitere Transportvorrichtung 46 aufweisen, die die sterilisierten Behälter von der Sterilisationsmaschine 10 übernimmt und aus dem Isolatorraum 48 transportiert.

Das erste Ventil 20 der zweiten Zuleitung 18 kann über die Signalverbindung 32 in diesem Ausführungsbeispiel mit der Steuereinheit 50 verbunden sein. Die Steuereinheit 50 kann dazu ausgebildet sein, das erste Ventil 20 bzw. dessen Aktuator zu steuern, um das erste Ventil 20 zu schalten, beispielsweise zu öffnen oder zu schließen. Damit kann die Steuereinheit 50 beim Wechsel der Sterilisationsmaschine 10 in den zweiten Zustand den Sterilisationsmittelstrom abstellen.

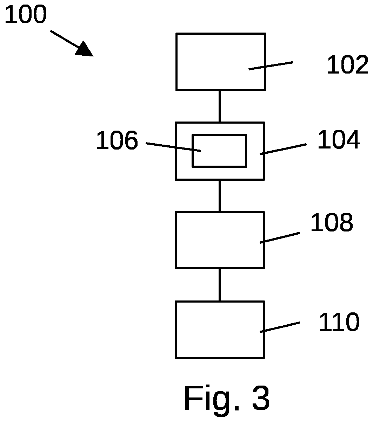

In Figur 3 ist ein Flussdiagramm des Verfahrens zum Steuern einer Sterilisationsmaschine 10 für Behälter dargestellt, wobei das Verfahren seiner Gesamtheit mit dem Bezugszeichen 100 referenziert wird.

Gemäß dem Verfahren 100 können in einem Schritt 102 der Sterilisationsmittelstrom und der Trägerfluidstrom in die Mischvorrichtung 14 eingeleitet werden, um das Sterilisationsgemisch zu erzeugen. Der Schritt 102 kann durchgeführt werden, wenn die Sterilisationsmaschine 10 im ersten Zustand ist oder aus einem anderen Zustand in den ersten Zustand wechseln soll.

In einem weiteren Schritt 104 kann der Sterilisationsmittelstrom für den Wechsel der Sterilisationsmaschine 10 in den zweiten Zustand abgeschaltet werden. Der Trägerfluidstrom strömt währenddessen weiter, d. h. der Trägerfluidstrom wird nicht abgeschaltet. Lediglich die Zugabe des Sterilisationsmittels zum Trägerfluid in der Mischvorrichtung 14 wird gestoppt.

Optional kann in einem Unter-Schritt 106 des Schritts 104 ein erstes Ventil 20 der Sterilisationsmaschine 10 zum Abschalten des Sterilisationsmittelstroms geschlossen werden. Alternativ kann eine Pumpe abgestellt werden, die den Sterilisationsmittelstrom erzeugt.

In einem weiteren Schritt 108 kann der Trägerfluidstrom im zweiten Zustand in eine Bypassleitung 34 eingeleitet werden, anstatt in die zu sterilisierenden Behälter zu strömen. Dazu können zum Beispiel die Ventile 36 und 38 derart geschaltet werden, dass der Trägerfluidstrom in die Bypassleitung 34 eingeleitet wird. Die Bypassleitung 34 kann derart angeordnet sein, dass der Trägerfluidstrom stromaufwärts vor der Sterilisationsmaschine 10 oder innerhalb der Sterilisationsmaschine 10 umgeleitet wird. Der Trägerfluidstrom kann weiter in einen die Sterilisationsmaschine 10 umgebenden Isolatorraum 48 umgeleitet werden. Die Umleitung des Trägerfluidstroms kann in allen vorgenannten Ausführungsbeispielen vorzugsweise vor dem Einleiten des Trägerfluidstroms in die Behälter erfolgen.

Alternativ oder zusätzlich kann in einem Schritt 110 eine Absaugung des Trägerfluidstroms erfolgen, bevor der Trägerfluidstrom in die Behälter strömt. Der Schritt 108 kann in beliebiger Reihenfolge mit dem Schritt 104 durchgeführt werden. Bevorzugt kann der Schritt 108 jedoch nach dem Schritt 104 oder gleichzeitig mit dem Schritt 104 durchgeführt werden. Gleiches gilt für den Schritt 110.

Die oben erläuterte Steuervorrichtung 28 und die oben erläuterte Steuereinheit 40 können dazu ausgebildet sein das oben erläuterte Verfahren 100 zum Steuern einer Sterilisationsmaschine für Behälter durchzuführen.

Das oben beschriebene Beispiel dient in keiner Weise einer Beschränkung der Erfindung. Vielmehr kann die Erfindung in vielfältiger Weise abgewandelt werden. Alle oben beschriebenen Merkmale der Erfindung können allein oder in Kombination miteinander wesentlich für die Erfindung sein.

### Bezugszeichenliste

- 10: Sterilisationsmaschine
- 12: Sterilisationsstation
- 14: Mischvorrichtung
- 16: erste Zuleitung
- 18: zweite Zuleitung
- 20: erstes Ventil
- 22: dritte Zuleitung
- 24: Transportrad
- 26: Verteilerstation
- 28: Steuervorrichtung
- 30: Signalverbindung
- 32: Signalverbindung
- 34: Bypassleitung
- 36: zweites Ventil
- 38: drittes Ventil
- 40: Anlage
- 42: Behälterherstellungsmaschine
- 44: Transportvorrichtung
- 46: Transportvorrichtung
- 48: Isolatorraum
- 50: Steuereinheit

## Patentansprüche

1. Verfahren (100) zum Steuern einer Sterilisationsmaschine (10) für Behälter, wobei die Sterilisationsmaschine (10) mindestens eine Mischvorrichtung (14) zum Erzeugen eines Sterilisationsgemisches aufweist und mindestens einen ersten Zustand, in dem das Sterilisationsgemisch in zu sterilisierende Behälter eingeleitet wird oder eingeleitet werden soll, und einen zweiten Zustand, in dem die Einleitung des Sterilisationsgemisches in die Behälter unterbrochen ist, umfasst, wobei im ersten Zustand ein Sterilisationsmittelstrom und ein Trägerfluidstrom zum Erzeugen des Sterilisationsgemisches in die Mischvorrichtung (14) eingeleitet (102) werden und das Sterilisationsgemisch den zu sterilisierenden Behältern zugeführt wird, **dadurch gekennzeichnet, dass** zum Wechseln in den zweiten Zustand der Sterilisationsmittelstrom abgeschaltet (104) wird, während der Trägerfluidstrom im zweiten Zustand weiterströmt.

2. Verfahren (100) nach Anspruch 1, **dadurch gekennzeichnet, dass** eine Temperatur, ein Druck und/oder ein Volumenstrom des Trägerfluidstroms im zweiten Zustand gegenüber dem ersten Zustand unverändert oder im Wesentlichen unverändert bleibt.

3. Verfahren (100) nach einem der vorhergehenden Ansprüche, **dadurch gekennzeichnet, dass** die Sterilisationsmaschine (10) mindestens ein Ventil (20) für das Einleiten des Sterilisationsmittelstroms aufweist, wobei das Ventil (20) beim Übergang in den zweiten Zustand geschlossen (106) wird.

4. Verfahren (100) nach einem der vorhergehenden Ansprüche, **dadurch gekennzeichnet, dass** der Sterilisationsmittelstrom Wasserstoffperoxid ist oder Wasserstoffperoxid aufweist.

5. Verfahren (100) nach einem der vorhergehenden Ansprüche, **dadurch gekennzeichnet, dass** der Trägerfluidstrom im zweiten Zustand anstatt in die zu sterilisierenden Behälter in eine Bypassleitung (34) eingeleitet (108) wird.

6. Verfahren (100) nach einem der vorhergehenden Ansprüche, **dadurch gekennzeichnet, dass** der Trägerfluidstrom im zweiten Zustand stromaufwärts vor der Sterilisationsmaschine (10) umgeleitet wird.

7. Verfahren (100) nach einem der Ansprüche 1 bis 5, **dadurch gekennzeichnet, dass** der Trägerfluidstrom im zweiten Zustand stromaufwärts vor dem Einleiten in die Behälter in der Sterilisationsmaschine (10) abgesaugt (110) wird.

8. Verfahren (100) nach einem der vorhergehenden Ansprüche, **dadurch gekennzeichnet, dass** der Trägerfluidstrom im zweiten Zustand in einen die Sterilisationsmaschine (10) umgebenden Isolatorraum (48) ausströmt.

9. Verfahren (100) nach einem der vorhergehenden Ansprüche, **dadurch gekennzeichnet, dass** der Trägerfluidstrom erwärmte Luft ist oder erwärmte Luft aufweist.

10. Sterilisationsmaschine (10) für Behälter, umfassend mindestens eine Mischvorrichtung (14) zum Erzeugen eines Sterilisationsgemisches und mindestens eine Steuervorrichtung (28) zum Steuern der Sterilisationsmaschine (10), wobei die Sterilisationsmaschine (10) mindestens einen ersten Zustand, in dem das Sterilisationsgemisch in zu sterilisierende Behälter eingeleitet wird oder eingeleitet werden soll, und einen zweiten Zustand, in dem die Einleitung des Sterilisationsgemisches in die Behälter unterbrochen ist, aufweist, **dadurch gekennzeichnet, dass** die Steuervorrichtung (28) zum Durchführen des Verfahrens (100) nach einem der vorhergehenden Ansprüche ausgebildet ist und der Sterilisationsmittelstrom im zweiten Zustand abgeschaltet ist, während der Trägerfluidstrom weiterströmt.

11. Sterilisationsmaschine (10) nach Anspruch 10, **dadurch gekennzeichnet, dass** die Sterilisationsmaschine (10) mindestens ein Ventil (20) zum Steuern des Sterilisationsmittelstroms aufweist, wobei die Steuervorrichtung (28) zum Übermitteln eines Steuersignals zum Schalten des Ventils (20) an einen Aktuator des Ventils (20) ausgebildet ist.

12. Anlage (40) zum Herstellen und/oder Befüllen von Behältern, umfassend mindestens eine Verarbeitungsmaschine (42), mindestens eine Transportvorrichtung (44), mindestens eine Steuereinheit (50) und mindestens eine Sterilisationsmaschine (10), wobei die Sterilisationsmaschine (10) mindestens eine Mischvorrichtung (14) zum Erzeugen eines Sterilisationsgemisches und mindestens einen ersten Zustand, in dem ein Sterilisationsgemisch in zu sterilisierende Behälter eingeleitet wird oder eingeleitet werden soll, und einen zweiten Zustand, in dem die Einleitung des Sterilisationsgemisches in die Behälter unterbrochen ist, aufweist, wobei die Transportvorrichtung (44) zum Transportieren von zu sterilisierenden Behältern von der Verarbeitungsmaschine (42) zur Sterilisationsmaschine (10) oder zum Transportieren von mittels der Sterilisationsmaschine (10) sterilisierter Behälter zu der Verarbeitungsmaschine (42) ausgebildet ist, **dadurch gekennzeichnet, dass** die Steuereinheit (50) zum Steuern zumindest der Sterilisationsmaschine (10) gemäß dem Verfahren (100) nach einem der Ansprüche 1 bis 9 ausgebildet ist und der Sterilisationsmittelstrom im zweiten Zustand abgeschaltet ist, während der Trägerfluidstrom weiterströmt.

13. Anlage (40) nach Anspruch 12, **dadurch gekennzeichnet, dass** die Verarbeitungsmaschine (42) eine Behälterherstellungsmaschine, insbesondere eine Blas- oder Streckblasmaschine, ist, wobei die Transportvorrichtung (44) die Behälter von der Verarbeitungsmaschine (42) zur Sterilisationsmaschine (10) transportiert.

14. Anlage (40) nach Anspruch 12, **dadurch gekennzeichnet, dass** die Verarbeitungsmaschine (42) eine Abfüllmaschine, insbesondere eine Getränkeabfüllmaschine, ist, wobei die Transportvorrichtung (44) die Behälter von der Sterilisationsmaschine (10) zur Verarbeitungsmaschine (42) transportiert.

15. Anlage (40) nach einem der Ansprüche 12 bis 14, **dadurch gekennzeichnet, dass** die Anlage (40) mindestens ein Ventil (20) für das Einleiten des Sterilisationsmittelstroms aufweist, wobei die Steuereinheit (50) zum Übermitteln eines Steuersignals zum Schalten des Ventils (20) an einen Aktuator des Ventils (20) ausgebildet ist.

16. Anlage (40) nach einem der Ansprüche 12 bis 15, **dadurch gekennzeichnet, dass** die Sterilisationsmaschine (10) in einem Isolatorraum (48) der Anlage (40) angeordnet ist.
